(19) Europäisches Patentamt
European Patent Office
Office européen des brevets

(11) Veröffentlichungsnummer : **0 124 084**
**B1**

(12) # EUROPÄISCHE PATENTSCHRIFT

(45) Veröffentlichungstag der Patentschrift : **07.09.88**

(51) Int. Cl.[4] : **C 07 C 76/02**, C 07 C 79/32

(21) Anmeldenummer : **84104681.6**

(22) Anmeldetag : **26.04.84**

(54) **Verfahren zur Herstellung von 6-Chlor-2,4-dinitrophenol.**

(30) Priorität : **30.04.83 DE 3315798**

(43) Veröffentlichungstag der Anmeldung : **07.11.84 Patentblatt 84/45**

(45) Bekanntmachung des Hinweises auf die Patenterteilung : **07.09.88 Patentblatt 88/36**

(84) Benannte Vertragsstaaten : **BE CH DE FR GB IT LI**

(56) Entgegenhaltungen : **DE-A- 2 212 848**

(73) Patentinhaber : **HOECHST AKTIENGESELLSCHAFT**
**Postfach 80 03 20**
**D-6230 Frankfurt am Main 80 (DE)**

(72) Erfinder : **Arndt, Otto, Dr.**
**Frankfurter Strasse 38**
**D-6238 Hofheim am Taunus (DE)**
Erfinder : **Papenfuhs, Theodor, Dr.**
**Heinrich-Bleicher-Strasse 40**
**D-6000 Frankfurt am Main 50 (DE)**

**EP 0 124 084 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft die Herstellung von 6-Chlor-2,4-dinitrophenol aus 2,4-Dinitrochlorbenzol und/oder 2,4-Dinitrophenylalkylethern der Formel

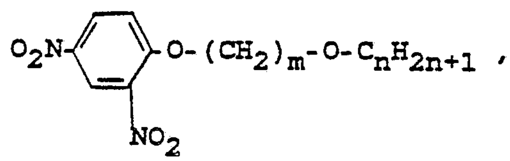

worin m die Zahl 2, 3 oder 4, und n die Zahl 1 oder 2 bedeuten, über die Stufe des 2,4-Dinitrophenols bzw. dessen Natrium- oder Kaliumsalzes (durch Verseifen) und Chlorieren der letztgenannten Verbindung.

Die beiden Stufen, Verseifen und Chlorieren, sind als Einszelschritte bekannt. So wird die Verseifung in « Grundlegende Operationen der Farbenchemie » von Fierz-David-Blangley, 8. Auflage (1952), Seite 329, und in BIOS Final Report Nr. 986, Item 22, Seite 183, und die Chlorierung in Beilstein 6, E II 247 sowie in Beilstein 6, 259 beschrieben. In der erstgenannten Beilstein-Literaturstelle heißt es, das 6-Chlor-2,4-dinitrophenol entstehe neben anderen Produkten bei der Einwirkung von Natriumhypochlorit auf 2,4-Dinitrophenol in verdünnter Salzsäure bei 15-20 °C. In der letztgenannten Beilstein-Literaturstelle heißt es, daß beim Einleiten von Chlor in ein siedendes Gemisch von Pikrinsäure, Wasser und Jod bis zum Auftreten von Chlorpikrin wenig 4-Chlor-2,6-dinitrophenol und viel 6-Chlor-2,4-dinitrophenol entsteht.

Wie ersichtlich erfolgen die zum Stand der Technik zählenden Chlorierungsverfahren, sei es mit elementarem Chlor oder mit Natriumhypchlorit, in stärker saurem Medium, wobei sich nennenswerte Mengen an Nebenprodukten bilden. Eigene Versuche einer Chlorierung mittels elementarem Chlor in konzentrierter Salzsäure führten zu keinem Erfolg, weil sich hierbei durch Austausch von Nitrogruppen gegen Chloratome 2,4-Dichlor-5-nitrophenol, 2,6-Dichlor-4-nitrophenol und eine Reihe unbekannter Verbindungen bilden. Eine Chlorierung mittels Natriumhypochlorit in wäßrigalkalischer Lösung führte ebenfalls nicht zum Ziel, weil hierbei keine Chlorierung stattfindet.

In FIAT 1313 I 100 wird eine andere Möglichkeit zur Herstellung von 6-Chlor-2,4-dinitrophenol beschrieben, wobei o-Chlorphenol sulfiert und die gebildete 2-Chlorphenolsulfonsäure-(4) unter Eliminierung der Sulfonsäuregruppe mit Salpetersäure zum 6-Chlor-2,4-dinitrophenol nitriert wird. Dieser Herstellungsweg ist wegen der damit verbundenen notwendigen Aufarbeitung der anfallenden Abfallsäuren unwirtschaftlich.

Es wurde nun gefunden, daß6-Chlor-2,4-dinitrophenol mit hoher Ausbeute und in hohem Reinheitsgrad durch Verseifen von 2,4-Dinitrochlorbenzol und/oder 2,4-Dinitrophenyl-alkylethern der Formel

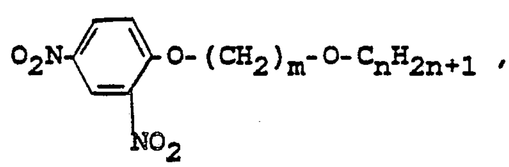

worin m die Zahl 2, 3 oder 4, und n die Zahl 1 oder 2 bedeuten, mit wäßriger Natrolauge oder Kalilauge und Chlorieren des erhaltenen 2,4-Dinitrophenols mit Natriumhypochlorit herstellen kann, indem man das2,4-Dinitrochlorbenzol und/oder die 2,4-Dinitrophenylalkylether mit verdünnter wäßriger Natronlauge oder Kalilauge im pH-Bereich 11 bis 13 verseift und das erhaltene 2,4-Dinitrophenol ohne vorausgehende Zwischenisolierung in wäßriger Suspension bei einem pH-Wert von 3,5 bis 7, vorzugsweise 4 bis 4,5 mit Chlorbleichlauge (wäßrige Lösung von Natriumhypochlorit und Natruimchlorid) bei Temperaturen von 5 bis 20 °C,vorzugsweise 8 bis 12 °C, chloriert, wobei das Natriumhypochlorit in äquivalenter Menge bis zu einem Überschuß von 20 Molprozent, bezogen auf das zu chlorierende Dinitrophenbol, angewendet wird.

Unter Chlorbleichlauge ist hierbei eine wäßrige Lösung von Natriumhypochlorit und Natriumchlorid zu verstehen, die durch Einleiten von Chlor in kalte wäßrige Natronlauge hergestellt werden kann. Die technische Chlorbleichlauge ist 13 gewichtsprozentig, was bedeutet, daß diese Chlorbleichlauge 150 g wirksames Chlor im Liter enthält. Neben dieser technischen Chlorbleichlauge, die beim Verfahren der vorliegenden Erfindung bevorzugt eingesetzt wird, können verfahrensgemäß auch Chlorbleichlaugen niedrigerer Konzentrationen verwendet werden. (Gmelins Handbuch der anorganichen Chemie 6 (1927), Seite 293 ; Ullmanns Encyklopädie der technischen Chemie 9, 4. Auflage (1975), Seite 544).

Nachdem die Chlorierung des 2,4-Dinitrophenols mittels Natriumhypochlorit in verdünnter Salzsäure gemäß der weiter oben zitierten Literaturstelle nicht zu einem reinen Produkt führt, und auch die Ausbeute zu wünschen übrig läßt, und eigene Versuche einer Chlorierung mittels Natriumhypochlorit weder in konzentrierter Salzsäure noch in alkalischem Medium zum Ziel führten, muß es als überraschend erachtet werden, daß die erfindungsgemäße Chlorierung mittels Natriumhypochlorit in dem genannten pH-Bereich (schwach sauer bis neutral) gelingt.

Nachstehend seien noch Einzelheiten bzw. bevorzugte Ausführungen des erfindungsgemäßen

Verfahrens beschrieben :

Was die Verseifung des 2,4-Dinitrochlorbenzols bzw. der 2,4-Dinitrophenylalkylether anbelangt, so empfiehlt es sich, die Verseifung mit der verdünnten wäßrigen Natronlauge oder Kalilauge im pH-Bereich 11-13 an der Luft oder in Gegenwart geringer Mengen eines Oxidationsmittels, wie Sauerstoff, Wasserstoffperoxid oder Natriumhypochlorit, vorzunehmen [vgl. hierzu HOUBEN-WEYL, VI/1c 1976, Seite 181 ; Deutsche Auslegeschrift 15 43 952 (1970), US-Patentschrift 3 283 011 (1962), J. Am. Chem. Soc. 95 (1973) 7, Seiten 2133-2136; Japanische Patentschrift 55/79 350 (1978) ; Japan Kokai Tokkyo Koho 8079, 350 (1980)].

Im Hinblick auf die auch oxidierende Wirkung des Natriumhypochlorits ist es zweckmäßig, diese Verbindung in äquivalenter Menge oder nur bis zu einem Überschuß von etwa 20 Molprozent, jeweils bezogen auf das zu chlorierende Dinitrophenol, anzuwenden. Die Anwendung größerer Überschüsse an Natriumhypochlorit führt zu Ausbeuteverlusten.

Vor der Zudosierung der Natriumhypochloritlösung zu der wäßrigen Suspension des angefallenen 2,4-Dinitrophenols ist es erforderlich, den bei der Verseifung des 2,4-Dinitrochlorbenzols bzw. der 2,4-Dinitrophenylalkylether zu den entsprechenden Natrium-bzw. Kaliumphenolaten angewandten Überschuß an Natronlauge bzw. Kalilauge mit Mineralsäure, beispielsweise Salzsäure, zu neutralisieren.

Hilfsmittel zur Förderung der Feinverteilung der Reaktionspartner, wie Tenside, oder Hilfsmittel zur Beschleunigung der Chlorierung, wie Eisen (III)-chlorid und Jod in Kombination, können der Reaktionsmischung zugesetzt werden.

Da bei der Chlorierung des 2,4-Dinitrophenols mit Natriumhypochlorit (NaOCl) ein Äquivalent Natriumhydroxid (NaOH) entsteht, empfiehlt es sich, die zur Neutralisation dieses freiwerdenden Äquivalents NaOH notwendige Menge Mineralsäure, beispielsweise Salzsäure, schon vor der Zudosierung der wäßrigen Natriumhypochloritlösung der Reaktionsmischung zuzugeben.Hierbei sinkt der pH etwa auf den Wert 3,5 ab, um dann während der Chlorierung wieder etwa auf den Wert 6-7 anzusteigen. Sollte der pH darüber hinaus ansteigen, so empfiehlt es sich, gleichzeitig mit dem letzten Anteil von Natriumhypochloritlösung Mineralsäure, beispielsweise Salzsäure, zuzugeben.

Beim erfindungsgemäßen Verfahren kann man vom 2,4-Dinitrochlorbenzol allein ausgehen. Man kann aber auch 2,4-Dinitrochlorbenzol in Mischung mit einem 2,4-Dinitrophenylalkylether zur Verseifung einsetzen, indem man das 2,4-Dinitrochlorbenzol zusammen mit dem 2,4-Dinitrophenylalkylether in wäßrige Natronlauge einträgt.

Die Möglichkeit, verfahrensgemäß eine Zwischenisolierung des bei der Verseifung gebildeten 2,4-Dinitrophenols umgehen zu können, ist im Hinblick auf die hohe Toxizität des 2,4-Dinitrophenols besonders wertvoll.

Schließlich kann dem 2,4-Dinitrochlorbenzol oder dem 2,4-Dinitrophenylalkylether bzw. der Mischung aus diesen beiden Verbindungen, zu Beginn oder nach Beendigung der Verseifungsreaktion 2,4-Dinitrophenol zugesetzt werden. Durch die letztgenannte Möglichkeit eignet sich das erfindungsgemäße Verfahren auch zur Aufarbeitung von Abfallprodukten, die 2,4-Dinitrophenol und/oder einen 2,4-Dinitrophenylalkylether, beispielsweise 2,4-Dinitromethoxyethoxybenzol, enthalten, wobei der Zusatz der 2,4-Dinitrophenylalkylether bzw. der Mischung aus 2,4-Dinitrophenol und den 2,4-Dinitrophenylalkylethern spätestens während der Verseifungsrekation erfolgen muß, und wobei gegebenenfalls auch die bereits bekannte, auf die Verseifung des 2,4-Dinitrochlorbenzols katalytisch wirkende Funktion von aliphatischen Hydroxyverbindungen zur Geltung kommen kann [J. Org. Chem. 43 (1978) 10, 1925-1929 ;J. Am. Chem. Soc. 98, 5663 (1976)].

6-Chlor-2,4-dinitrophenol ist ein Zwischenprodukt für Farbstoffe. Die OH-Gruppe dieser Verbindung läßt sich beispielsweise gegen Chlor austauschen unter Bildung des 1,2-Dichlor-3,5-dinitrobenzols (DE-OS 2 001 570 (1970), B. 44 (1911) S. 3 730, CA 63 (1965) 6 898 h). Das stark aktivierte Chloratom in 2-Stellung der so erhaltenen Verbindung läßt sich gegen eine ganze Reihe von Nucleophilen austauschen, beispielsweise durch Cyanamid (Europäische Patentanmeldung 53 714), wodurch man schließlich zum entsprechenden 5-Amino-benzimidazolon (US-Patentschrift 4 246 196) gelangt. Aber auch ein direkter Austausch der OH-Gruppe im 6-Chlor-2,4-dinitrophenol gegen Stickstoffnucleophile ist möglich, beispielsweise durch eine Aminogruppe durch Behandlung mit Ammoniak unter Druck zum entsprechenden 6-Chlor-2,4-dinitranilin (HOUBEN-WEYL, VI/1c 2 (1976), 1 160), welches eine Kupplungskomponente für die Herstellung einer Reihe von Dispersionsazofarbstoffen darstellt (DE-OS 2 155 866, DE-OS 2 256 314,Japanische bekanntgemachte Patentanmeldung 72/33 481 und Japanische Offenlegungsschrift 72/26 417).

Die nachfolgenden Beispiele dienen zur Erläuterung des erfindungsgemäßen Verfahrens, ohne es darauf zu beschränken. Teile bedeuten Gewichtsteile.

## Beispiel 1

203 Teile 2,4-Dinitrochlorbenzol (Erstarrungspunkt 49 °C) werden in einer Mischung aus 303 Teilen 33 %iger Natronlauge und 3 000 Teilen Wasser bei 95 ° C unter Luftatmosphäre zum 2,4-Dinitrophenol verseift. Die erhaltene Suspension, deren pH-Wert 13 beträgt, wird durch Zusatz von 185 Teilen 31 %iger Salzsäure auf den pH-Wert 3,5 gestellt. Nach Zugabe von 10 Teilen Naphthalinsulfosäure-Formaldehyd-Kondensat (Natriumsalz) (Dispergiermittel),1 Teil Eisen (III)-chlorid und 1 Teil Jodtrichlorid werden 600

Teile 13 gewichtsprozentige Chlorbleichlauge (enthaltend 150 g wirksames Chlor pro Liter) innerhalb von 8 Stunden bei 10 °C zugetropft, wobei der pH ansteigt. im Laufe der Zugabe erreicht der pH den Wert 6. Nach Erreichen dieses Wertes wird durch gleichzeitiges Zutropfen von 5-20 Teilen 31 %iger Salzsäure während des Zutropfens der restlichen Chlorbleichlauge bewirkt, daß der pH bei diesem Wert (pH 6) gehalten wird. Anschließend wird die Suspension mittels 170 Teilen 31 %iger Salzsäure auf den pH-Wert 1,0 gestellt und geringe Anteile von noch vorhandenem Natriumhypochlorit werden mit 15 Teilen 40 %iger Natriumhydrogensulfitlösung zerstört. Dann wird das erhaltene Produkt durch Abfiltrieren bei etwa 10 °C isoliert. Man erhält 200 Teile hellgelbes 6-Chlor-2,4-dinitrophenol (Fp : 104-107 °C) mit einem Gehalt von maximal etwa 5 % 2,4-Dinitrophenol. Dünnschichtchromatographisch sind keine Nebenprodukte nachweisbar.

Die Ausbeute an 6-Chlor-2,4-Dinitrochlorphenol beträgt 91 % der Theorie

Beispiel 2

Ein Gemisch aus 183 Teilen 2,4-Dinitrochlorbenzol, 16 Teilen 2,4-Dinitrophenol und 3 Teilen 2,4-Dinitromethoxyethoxybenzol wird in eine Mischung aus 303 Teilen 33 %iger Natronlauge und 3 000 Teilen Wasser bei 95 °C unter Luftatmosphäre zum 2,4-Dinitrophenol verseift. Die erhaltene Suspension wird durch Zusatz von 185 Teilen 31 %iger Salzsäure auf den pH-Wert 3,5 gestellt. Nach Zugabe von 10 Teilen Naphthalinsulfosäure-Formaldehyd-Kondensat (Natriumsalz) (Dispergiermitel), 1 Teil Eisen (III)-chlorid und 1 Teil Jodtrichlorid werden 650 Teile 13 gewichtsprozentiger Chlorbleichlauge (enthaltend 150 g wirksames Chlor pro Liter) innerhalb von 8 Stunden bei 10 °C zugetropft, wobei der pH ansteigt. Im Laufe der Zugabe erreicht der pH den Wert 4,5. Nach Erreichen dieses Wertes wird durch gleichzeitiges Zutropfen von 36 Teilen 31 %iger Salzsäure während des Zutropfens der restlichen Chlorbleichlauge bewirkt, daß der pH während der Chlorierung im Bereich 4-4,5 gehalten wird. Anschließend wird die Suspension mittels 170 Teilen 31 %iger Salzsäure auf den pH-Wert 1,0 gestellt, und geringe Anteile von noch vorhandenem Natriumhypochlorit werden mit 15 Teilen 40 %iger Natriumhydrogensulfitlösung zerstört. Dann wird das erhaltene Produkt durch Abfiltrieren bei etwa 10 ° C isoliert. Man erhält 200 Teile hellgelbes 6-Chlor-2,4-dinitrophenol (Fp : 101-109 °C), welches nur noch Spuren von 2,4-Dinitrophenol (0,5 %) enthält.

Die Ausbeute beträgt 91 %der Theorie.

Beispiel 3

Verwendet man anstelle von 203 Teilen 2,4-Dinitrochlorbenzol ein Gemisch aus 183 Teilen 2,4-Dinitrochlorbenzol, 16 Teilen 2,4-Dinitrophenol und 3 Teilen 2,4-Dinitromethoxyethoxybenzol und verfährt im übrigen wie in Beispiel 1 beschrieben, so erhält man 205 Teile 6-Chlor-2,4-dinitrophenol (Fp : 104-107 °C) mit einem Gehalt von maximal 5 % 2,4-Dinitrophenol.

Die Ausbeute beträgt 93 % der Theorie.

Beispiel 4 (Vergleichsbeispiel)

Verwendet man anstelle von 600 Teilen 13 gewichtsprozentiger Chlorbleichlauge 830 Teile 13-gewichtsprozentiger Chlorbleichlauge (enthaltend 150 g aktives Chlor pro Liter) und verfährt im übrigen wie in Beispiel 1 beschrieben, so erhält man nur 186 Teile 6-Chlor-2,4-dinitrophenol (Fp : 102-105 °C), was einer Ausbeute von 85 % der Theorie entspricht.

Beispiel 5 (Vergleichsbeispiel)

Verwendet man anstelle von 600 Teilen 13-gewichtsprozentiger Chlorbleichlauge 1 308 Teile 13-gewichtsprozentiger Chlorbleichlauge (enthaltend 150 g wirksames Chlor pro Liter) und verfährt im übrigen wie in Beispiel 1 beschrieben, so erhält man nur 170 Teile 6-Chlor-2,4-dinitrophenol (Fp : 104-107 °C), was einer Ausbeute von 77 % der Theorie entspricht. Der Gehalt an 2,4-Dinitrophenol beträgt maximal etwa 5 %.

Beispiel 6

Verfährt man gemäß Beispiel 1, jedoch mit dem Unterschied, daß man die Verseifung des 2,4-Dinitrochlorbenzols nicht nur unter Luftatmosphäre (Oxidationsmittel), sondern zusätzlich mittels 55 Teilen 13-gewichtsprozentiger Chlorbleichlauge (Oxidationsmittel) bzw. durch zusätzliches Einblasen $CO_2$-freier Luft (Oxidationsmittel) durchführt, so erhält man das 6-Chlor-2,4-dinitrophenol in gleicher Ausbeute und in gleichem Reinheitsgrad wie gemäß Beispiel 1.

Beispiel 7 (Vergleichsbeispiel)

203 Teile 2,4-Dinitrochlorbenzol (Erstarrungspunkt 49 °C) werden in einer Mischung aus 303 Teilen

33 %iger Natronlauge und 200 Teilen Wasser bei 95 °C unter Stickstoffatmosphäre zum 2,4-Dinitrophenol verseift. Die erhaltene Suspension wird durch Zusatz von 185 Teilen 31 %iger Salzsäure auf den pH-Wert 3,5 gestellt. Nach Zugabe von 10 Teilen Naphthalinsulfonsäure-Formaldehyd-Kondensat (Natriumsalz) (Dispergiermittel), 1 Teil Eisen (III)-chlorid und 1 Teil Jodtrichlorid werden 650 Teile 13-gewichtsprozentiger Chlorbleichlauge (enthaltend 150 Teile wirksames Chlor pro Liter) innerhalb von 8 Stunden bei 10 °C zugetropft, wobei der pH ansteigt. Gegen Ende der Zugabe erreicht der pH den Wert 6. Nach Erreichen dieses Wertes wird durch gleichzeitiges Zutropfen von 5-20 Teilen 31 %iger Salzsäure und restlicher Chlorbleichlauge der pH bei diesem Wert (pH 6) gehalten. Anschließend wird die Suspension durch Zugabe von 170 Teilen 31 %iger Salzsäure auf den pH-Wert 1,0 gestellt und geringe Anteile von noch vorhandenem Natriumhypochlorit werden mittels 15 Teilen 40 %iger Natriumhydrogensulfitlösung zerstört. Dann wird das erhaltene Produkt durch Abfiltrieren bei etwa 10 °C isoliert. Man erhält nur 130 Teile 6-Chlor-2,4-dinitrophenol von schwarzbraunem Aussehen und einen im Dünnschichtchromatogramm deutlich nachweisbaren Gehalt an nicht verseiftem 2,4-Dinitrochlorbenzol.

**Patentansprüche**

1. Verfahren zur Herstellung von 6-Chlor-2,4-dinitrophenol mit hoher Ausbeute und in hohem Reinheitsgrad durch Verseifen von 2,4-Dinitrochlorbenzol und/oder 2,4-Dinitrophenylalkylethern der Formel

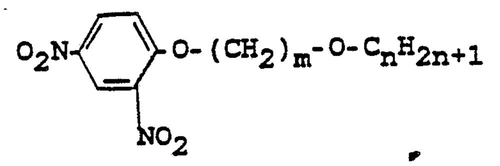

worin m die Zahl 2, 3 oder 4, und n die Zahl 1 oder 2 bedeuten, mit wäßriger Natronlauge oder Kalilauge und Chlorieren des erhaltenen 2,4-Dinitrophenols mit Natriumhypochlorit, dadurch gekennzeichnet, daß man das 2,4-Dinitrochlorbenzol und/oder die 2,4-Dinitrophenylalkylether mit verdünnter wäßriger Natronlauge oder Kalilauge im pH-Bereich 11 bis 13 verseift und das erhalten 2,4-Dinitrophenol ohne vorausgehende Zwischenisolierung in wäßriger Suspension bei einem pH-Wert von 3,5 bis 7 mit Chlorbleichlauge (wäßrige Lösung von Natriumhypochlorit und Natriumchlorid) bei Temperaturen von 5 bis 20 °C chloriert, wobei das Natriumhypochlorit in äquivalenter Menge bis zu einem Überschuß von 20 Molprozent, bezogen auf das zu chlorierende Dinitrophenol, angewendet wird.

2. Verfahren nach Anspruch 1, dadurch gekennzeichnet, daß man mit einer 13-gewichtsprozentigen Chlorbleichlauge (enthaltend 150 g wirksames Chlor im Liter) bei einem pH-Wert von 4-4,5 bei Temperaturen von 8-12 °C chloriert.

**Claims**

1. A process for the preparation of 6-chloro-2,4-dinitrophenol in a high yield and in a high degree of purity by saponifying 2,4-dinitrochlorobenzene and/or 2,4-dinitrophenyl alkyl ethers of the formula

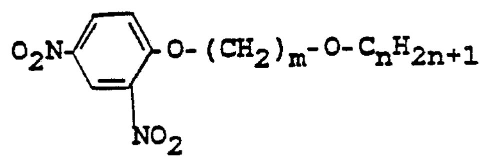

in which m denotes the number 2, 3 or 4 and n denotes the number 1 or 2, by means of aqueous sodium hydroxide or potassium hydroxide solution and chlorinating the resulting 2,4-dinitrophenol with sodium hypochlorite,which comprises saponifying the 2,4-dinitrochlorobenzene and/or the 2,4-dinitrophenyl alkyl ethers by means of dilute aqueous sodium hydroxide or potassium hydroxide solution within the pH range from 11 to 13 and chlorinating the 2,4-dinitrophenol obtained, without prior isolation as an intermediate, in aqueous suspension at a pH value of 3.5-7 by means of chlorine bleach liquor (aqueous solution of sodium hypochlorite and sodium chloride) at temperatures of 5 to 20 °C, the sodium hypochlorite being employed in an equivalent amount up to an excess of 20 mole percent, relative to the dinitrophenol to be chlorinated.

2. The process as claimed in claim 1, wherein chlorination is carried out with a 13 percent strength by weight chlorine bleach liquor (containing 150 g of active chlorine per liter) at a pH value of 4-4.5 and at temperatures of 8-12 °C.

**Revendications**

1. Procédé pour préparer le chloro-6 dinitro-2,4 phénol à l'état très pur et avec un rendement élevé, par saponification du dinitro-2,4 chlorobenzène et/ou d'éthers alkyliques du dinitro-2,4 phénol répondant à la formule :

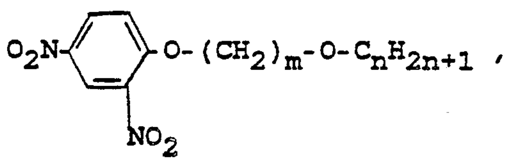

dans laquelle m est égal à 2, à 3 ou à 4 et n est égal à 1 ou à 2, au moyen d'une solution aqueuse d'hydroxyde de sodium ou d'hydroxyde de potassium, et chloration du dinitro-2,4 phénol obtenu au moyen d'hypochlorite de sodium, procédé caractérisé en ce qu'on saponifie le dinitro-2,4 chlorobenzène et/ou les éthers alkyliques du dinitro-2,4 phénol par une solution aqueuse diluée d'hydroxyde de sodium ou d'hydroxyde de potassium dans l'intervalle de pH allant de 11 à 13, et on chlore le dinitro-2,4 phénol obtenu, sans le séparer intermédiairement, en suspension aqueuse, à un pH de 3,5 à 7, au moyen d'une lessive de blanchiment au chlore (solution aqueuse d'hypochlorite de sodium et de chlorure de sodium) à des températures de 5 à 20 °C, l'hypochlorite de sodium étant alors utilisé en une quantité comprise entre la quantité équivalente et un excès d'au plus 20 % en moles par rapport au dinitrophénol à chlorer.

2. Procédé selon la revendication 1 caractérisé en ce qu'on effectue la chloration au moyen d'une lessive de blanchiment au chlore à 13 % en poids (contenant 150 g de chlore actif par litre), à un pH de 4 à 4,5 et à des températures de 8 à 12 °C.